# EUROPEAN PATENT SPECIFICATION

(11) **EP 1 747 793 B1**
(45) Date of publication and mention of the grant of the patent: **20.01.2010**
(21) Application number: 06253924.2
(22) Date of filing: 27.07.2006
(51) Int. Cl.: A61M 25/00

(54) **Methods of making catheter shaft tubes**
Methoden zur Herstellung von Katheterschaftröhren
Méthodes de fabrication de tubes pour cathéter

(30) Priority: 27.07.2005 US 191636
(43) Date of publication of application: 31.01.2007
(73) Proprietor: Cordis Neurovascular, Inc., Miami Lakes, Florida 33014 (US)
(72) Inventor: Sherman, Darren R., Florida 33301 (US); Slazas, Robert R., Miami Florida 33176 (US)
(74) Representative: Gover, Richard Paul

(56) References cited:
- EP-A- 1 611 914
- EP-A2- 0 807 446
- WO-A2-95/18647
- US-A1- 2003 028 173
- US-A1- 2003 060 757
- US-A1- 2004 207 127

## Description

The present invention relates to methods of making catheters.

Many catheters have a relatively long and flexible tubular shaft defining one or more passages or "lumens." The end of the catheter that is advanced to a desired site for treatment is customarily referred to as the "distal" end, while the other end is called the "proximal" end. The proximal end of the shaft is generally coupled to a hub for ease of use by a physician. The hub may define one or more proximal ports for communication with the lumen(s) defined by the catheter shaft, and may have a luer-lock fastener or some other means for connecting the catheter to other medical devices or systems.

Some catheter shafts are made of one or more tubular shaft members, each of which may include any of (he following features, or any combination thereof: one or more polymer layers, one or more types of reinforcing members (such as for example coils, fibers, mesh or braids), one or more stiffening members or wires, and/or a hypotube.

In those catheter shaft tubes with more than one polymer layer, and also some kind of reinforcement extending between the polymer layers, it may be desirable to select different materials for inner and outer layer(s). For example, a catheter designer might select a lubricious material for an inner layer and stronger material(s) for an outer layer(s). This type of multi-layer design allows greater performance and optimizing various desired properties.

Because most catheter shaft tubes are of relatively small diameter, the multiple polymer layers are generally assembled from the inner layer outward. Each polymer layer is usually applied to a core wire or mandrel, whether by extrusion, heat-shrinking or other methods, in an operation conducted at or above the melting temperature of the polymer material of that layer. However, if an outer layer is applied to an existing inner layer, and the outer layer melting temperature is greater than that of the inner layer, then the excessive temperature may cause manufacturing difficulties. For example, the inner layer may melt, run, reform, or change thickness in some areas. Another factor is that catheter shaft tubes are generally built up around a metallic core wire or mandrel, which may act as a heat sink during heating operations.

As a result, the selection of polymer materials available for all but an innermost layer is limited to polymers having melting temperatures equal to or less than that of the innermost layer.

It would be desirable to be able to select materials for the polymer layers based on performance characteristics and material properties such as strength, flexibility and lubricity, without being limited to outer layers with successively increasing melting temperatures. In other words, it would be desirable to provide tubular members for use in a catheter shaft, of which at least a portion has the following features: at least one inner and outer polymer layer, with reinforcement between the inner and outer layers, in which an outer layer has a higher melting temperature than an inner layer.

Such a construction would allow the catheter designer to select additional polymer materials for the outer layer(s) of a catheter tube for advantageous performance properties, rather than being limited to materials having low melting temperatures.

This disclosure of the present invention will include various possible features and embodiments. However, the present invention scope as set forth in each of the claims and is not limited to the particular arrangements described in this disclosure.

### Therapies:

Catheters are used in a variety of therapeutic applications, including intravascular catheters for procedures such as angioplasty and/or deploying medical devices such as stents. Approximately one million angioplasties are performed worldwide each year to treat vascular disease, including coronary, peripheral and neurological blood vessels partially or totally blocked or narrowed by a lesion, stenosis, thrombosis, and/or vasospasm.

By way of example, the present invention will be described in relation to vascular treatments, including coronary, peripheral and neurological angiography and angioplasty. However, it should be understood that the present invention relates to any claimed method of making such a catheter and/or shaft and is not limited to vascular catheters, angiography, angioplasty, or stents, or even use in blood vessels.

Common treatment methods for using an angiography or angioplasty catheter include advancing a guidewire into the body of a patient, by directing the guidewire distal end percutaneously through an incision and along a body passage until it is located within or beyond the desired site. The term "desired site" refers to the location in the patient's body currently selected for treatment by a health care professional. When the guidewire is within the catheter guidewire lumen, the catheter may be advanced or withdrawn along a path defined by the guidewire.

In the case of a balloon catheter, after the balloon is disposed within the desired site, it can be selectively inflated to press outward on the body passage at relatively high pressure to a relatively constant diameter, in the case of an inelastic or non-compliant balloon material. This outward pressing of a constriction or narrowing at the desired site in a body passage is intended to partially or completely re-open or dilate that body passageway or lumen, increasing its inner diameter or cross-sectional area. In the case of a blood vessel, this procedure is referred to as angioplasty. The objective of this procedure is to increase the inner diameter or cross-sectional area of the vessel passage or lumen through which blood flows, to encourage greater blood flow through the newly expanded vessel. The narrowing of the body passageway lumen is called a lesion or stenosis, and may be formed of hard plaque or viscous thrombus.

Some catheters are used to deliver and deploy stents or other medical devices, in a manner generally known in the art. Stents, for example, are generally tubular scaffolds for holding a vessel or body passage open.

### Catheter Performance:

EP-1611914 discloses a balloon catheter shaft design for medical treatment of a patient. The balloon catheter allows for further therapeutic dilation or deployment of medical devices such as stents or grafts. At least a potion of the shaft has an inner tubular body defining at least a portion of a guidewire lumen surrounded by an outer tubular body defining at least a portion of an inflation lumen. The proximal portion of the inner body is reinforced by a hypotube. A distal end of the hypotube provides a graduated flexibility transition with a distal spiral-cut segment, in which the pitch of the spiral-cut pattern decreases to provide increase in flexibility.

WO-95/18647 discloses a thermoplastic polyimide balloon catheter. The catheter construction may either be integral or unitary in which the shaft or portion thereof and the balloon are manufactured as a single unit. Alternatively, the construction may be comprised of a separate shaft to which a balloon is attached by adhesive or other bonding.

US-2003/0028173 discloses an intravascular catheter including a reinforcement layer having a plurality of interwoven metal wire members and polymer members. The polymer members are thermally processed to permeate the metal wire members to form a polymeric layer having an orientation which is interwoven with the metal wire members.

US-2004/0207127 discloses a method of producing laminated inflatable, substantially inextensible expander members having composite properties enhancing their use on intravascular catheters, such as angioplasty catheters. Polymeric compounds of different properties are coextruded to create a multi layer parison. The parison is subsequently drawn and expanded in a blow molding operation to yield an expander member exhibiting enhanced properties.

EP-0807446 discloses a catheter having an elongated tubular body with a distal end, a proximal end and at least one lumen extending therebetween. The body is made from three layers of extruded plastic which adhere to each other. The body has inner and outer layers made from a polymer, and a middle layer also made from a polymer. The middle layer is more flexible and has a lower melt temperature than the inner and the outer layers.

US 6,858,204 B1 discloses a guiding catheter for use in coronary angioplasty and other cardiovascular interventions which incorporates a plurality of segment of selected flexural modulus in the shaft of the device. The segments which have a different flexibility than the sections immediately proximal and distal to them, creating zones in the catheter shaft which are either more or less flexible than other zones of the shaft. The flexibility and length of the shaft in a given zone is then matched to its clinical function and role. Also disclosed is a process of manufacturing the catheter using a mandrel on which an inner layer, a support layer and an outer layer are applied by extrusion.

It is desirable to provide a catheter having an optimum combination of various performance characteristics, which may be selected among: flexibility, lubricity, pushability, trackability, crossability, low profile and others. Flexibility may relate to bending stiffness of a medical device (catheter and/or stent, for example) in a particular region or over its entire length, or may relate to the material hardness of the components. Lubricity may refer to reducing friction by using low-friction materials or coatings. Pushability may relate to the column strength of a device or system along a selected path. Trackability may refer to a capability of a device to successfully follow a desired path, for example without prolapse. Crossability may be clarified by understanding that physicians prefer to reach the desired site with the catheter while encountering little or no friction or resistance. Profile may refer to a maximum lateral dimension of the catheter, at any point along its length.

Catheter tubes fabricated according to embodiments of the present invention provide various advantages, which may include: strength, flexibility and smooth transitions thereof, lubricity including a lubricious guidewire lumen, reinforcement, pushability, optimized flexibility along the length of the catheter shaft, torsional strength, pull strength, low profile, etc.

The scope of the present invention is defined by the appended claims.

According to a first embodiment of the present invention a catheter may be fabricated by a method of making a tubular body for a catheter shaft, comprising the steps of:
a. providing a core wire;
b. coating the core wire with a thermally insulating material;
c. applying an inner polymer layer having an inner melting temperature onto the coated core wire;
d. applying one or more reinforcing members to an outer surface of the inner polymer layer; and
e. applying an outer polymer layer around an outer surface of the inner polymer layer and reinforcement member; the outer polymer layer having an outer melting temperature that is higher than the inner melting temperature; wherein the inner and outer polymer layers and the reinforcement form a tubular assembly around the coated core wire;
f. releasing and removing the coated core wire from inside the inner polymer layer, such that the remainder forms a catheter shaft tube.

Embodiments of the invention will now be described by way of example with reference to the accompanying drawings, in which:
Figure 1 is an external perspective view of a catheter;
Figure 2 is an external perspective view of a balloon catheter;
Figures 3-6 are longitudinal cross-section views of different catheter tubes, each having reinforcement extending between at least two polymer layers;
Figures 7-12 are transverse cross-section views of various steps in processes for making catheter tubes according to the principles of the present invention;
Figures 11A and 11B are partial transverse cross-section views of some different possible arrangements during the step shown in Figure 11;

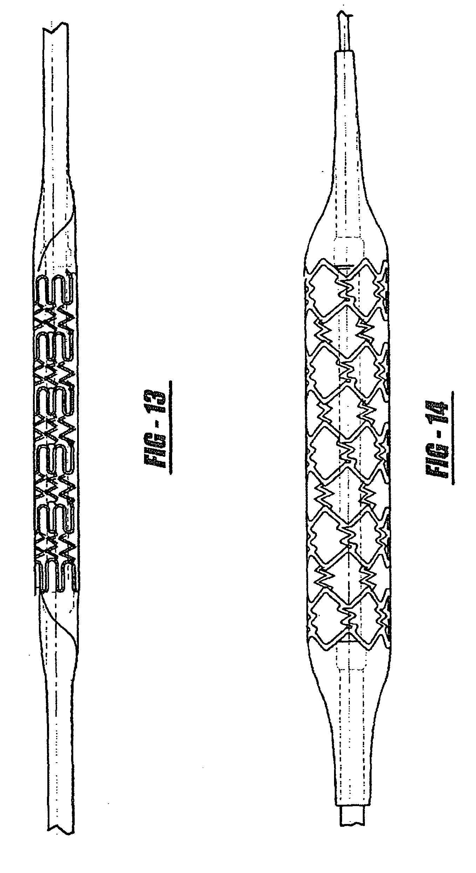

Figures 13 and 14 are partial side elevation views of a balloon catheter stent delivery system, in deflated and inflated states; and
Figure 15 is a diagrammatic flow chart of a process for making catheter tubes according to the principles of the present invention.

The following description of the preferred embodiments of the present invention is merely illustrative in nature, and as such it does not limit in any way the present invention, its application, or uses. Numerous modifications may be made by those skilled in the art without departing from the scope of the invention, which is defined by the appended claims.

### Catheters:

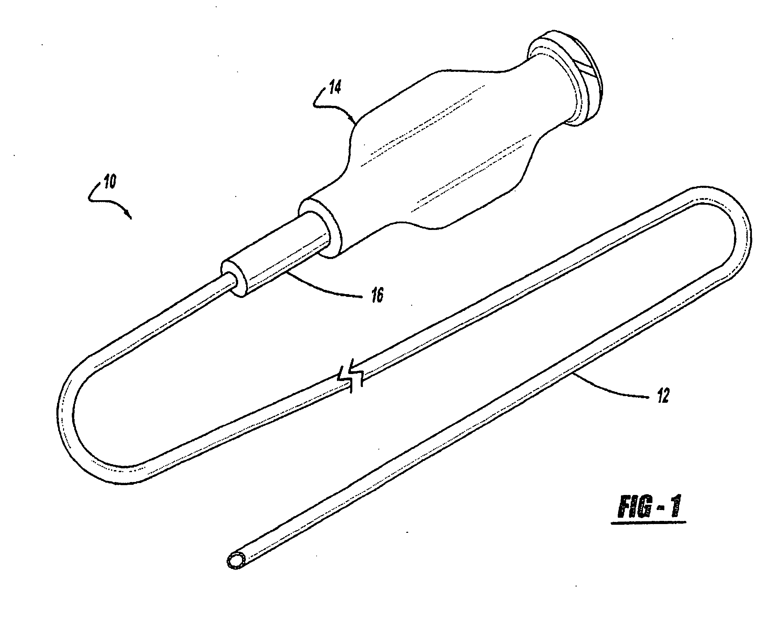

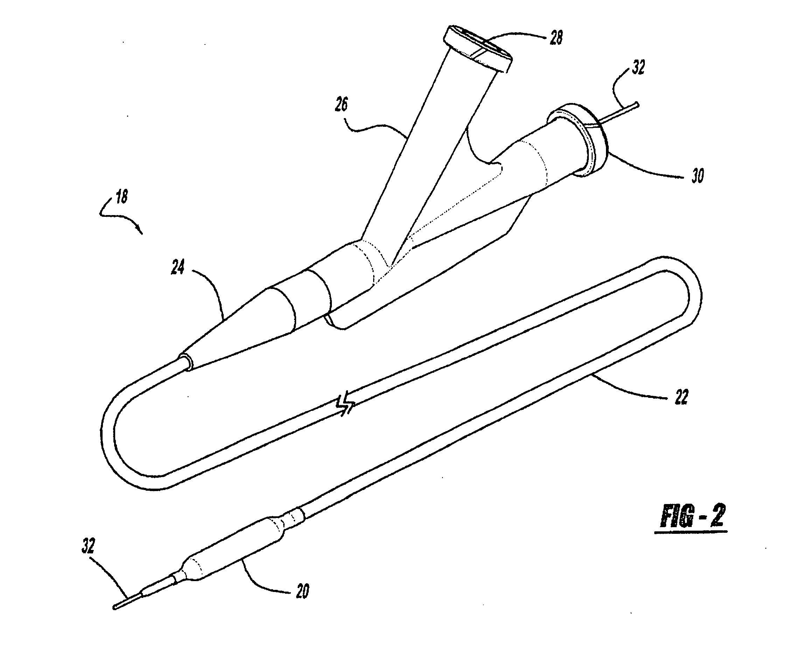

Generally, the present examples have to do with catheters. and tubes for use in catheter shafts, the present invention has to do with methods of making such tubes and catheters. Referring to the drawings, some examples of catheters are shown in Figures 1 and 2. Of course, they are only two possible examples of the various types of catheters with shafts having tubes that may be made according to the principles of the present invention.

The catheter 10 of Figure 1 has a relatively long and flexible tubular shaft 12, a hub 14, and a flexible strain relief 16. The shaft 12 extends from a distal end to a proximal end. where the hub 14 and strain relief 16 are affixed to the shaft 12. The catheter 18 of Figure 2 has an inflatable balloon 20, a relatively long and flexible tubular shaft 22, a strain relief 24, and a hub 26 defining an inflation port 28 and a guidewire port 30. The balloon 20 is affixed to the shaft 22 near its distal end, and the hub 26 and strain relief 24 are affixed to the shaft proximal end. Either or both catheters of Figures 1 and 2 may be used with a guidewire 32.

Catheter 10 defines a passage or lumen extending inside the tubular member(s) of its shaft 12, which may be used as an infusion or guidewire lumen, for example. Catheter 18 defines at least two lumens, one of which is an inflation lumen connecting the balloon interior with the inflation port for selectively inflating and deflating the balloon. A second lumen defined by catheter 18 is a guidewire lumen is adapted to receive an elongated flexible guidewire in a sliding fashion. The guidewire and catheter 18 may thus be advanced or withdrawn independently, or the catheter 18 may be guided along a path selected with the guidewire.

### Tubes:

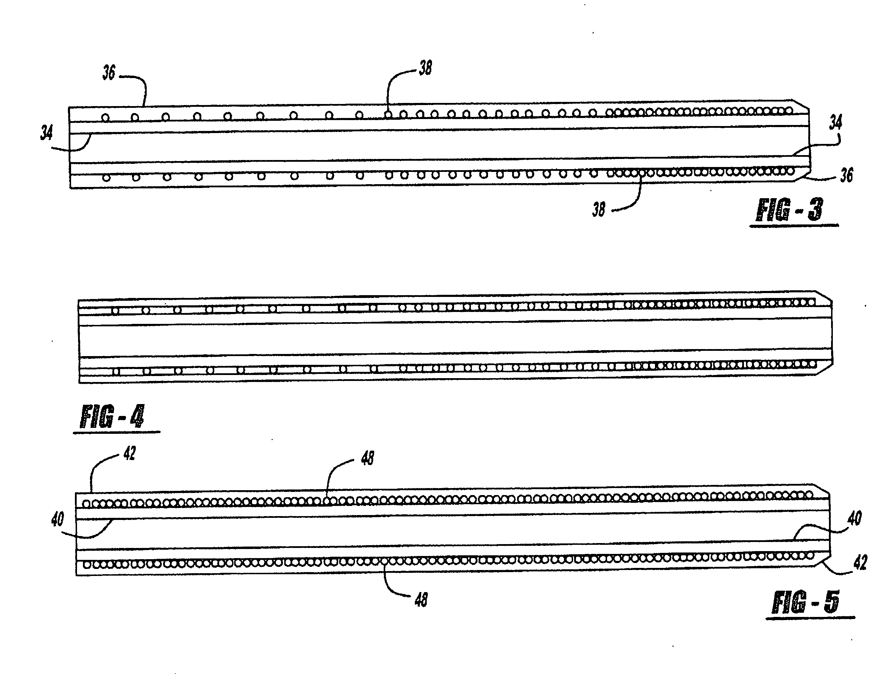

Some examples of possible designs for tubular bodies are shown in Figures 3-6. all of which have at least two polymer layers and some type(s) of reinforcement extending between the layers.

Figure 3 shows a catheter shaft tube having an inner and outer polymer layer 34 and 36, and a coil or braid 38. The inner layer 34 may be a lubricious polymer material, such as high density polyethylene (HDPE) or polytetrafluoroethylene (PTFE). The outer layer 36 is a strong polymer material, which is may be selected to have various other desirable performance properties. For example, the outer layer polymer may be selected for flexibility and strength, and to bond well with the material(s) selected for other components, such as a hub and/or balloon. Examples of acceptable materials include polyamides such as nylons, or polyether block amide (PEBA).

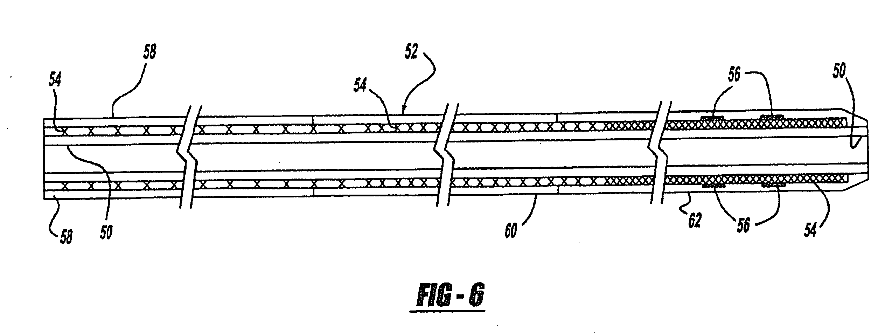

Similarly, the catheter shaft tube of Figure 5 also has an inner and outer polymer layer 40 and 42. The catheter shaft tubes of Figures 4 and 6 show at least an inner and outer polymer layer, and may include a third intermediate layer as well.

In addition, the specific examples shown in Figures 3-6 also have internal reinforcement in the form of a braid or coil. The reinforcement is depicted in a diagrammatic manner for clarity, and may be one or more wires coiled around the catheter shaft tube, between the inner and outer layers. If more than one wire is used, they may be arranged in a criss-crossing fashion. The wires may be a metal such as stainless steel or nitinol, or another strong material such as an aramid fiber, for example that sold under the trade name Kevlar, or a synthetic fiber based on ultra high molecular weight polyethylene, for example that sold under the tradename Dyneema.

In the example of Figure 5, the reinforcement wire(s) 48 is arranged with a pitch angle that is constant along the length of the catheter shaft tube. In Figures 3, 4 and 6. the wires are arranged with a pitch angle that decreases in the distal direction, in other words, the wraps of the wires are closer together near the distal end of tube than at the proximal end. This decreasing pitch, measured in increasing wires per inch ("pics"), may be arranged progressively along the length of the inner body, in linear or non-linear fashion, or in specific segments.

The specific example shown in Figure 6 has an inner and outer polymer layer 50 and 52, reinforcement 54, and a pair of radiopaque markers 56. Also, the outer polymer layer has multiple segments of differing flexibility. Specifically, outer layer 52 has a proximal, middle, and distal segment 58, 60 and 62, arranged in order of increasing flexibility from the proximal to the distal direction. If the reinforcement pitch angles change along the length of the tube, it is also possible to arrange the pitch angle segments to align with the segments of different flexibility of the outer layer material but need not be so aligned, as shown in Figure 6.

The inner surface of catheter shaft tubes may be of a material selected for high lubricity, for example which will present low frictional resistance to movement of a guidewire inserted within the lumen. Some catheters have used an inner layer defining a guidewire lumen that is made of Teflon (PTFE), and it is possible to likewise use PTFE in a catheter according to the present invention.

Another possibility is to use a different material for the guidewire lumen. Because many guidewires have a PTFE coating, in some operating conditions, it is possible that the resulting interface between similar materials, PTFE tube on PTFE-coated guidewire. to exhibit a slight "slip stiction" effect. Accordingly, another lubricant material may be used, for example HDPE, as the inner layer of inner body.

One or more radiopaque markers may be provided, to indicate the position of portion(s) of the catheter to a physician using x-ray video. In the example of Figure 6, a pair of marker bands made of a radiopaque material are provided near the distal end of the catheter shaft tube. The radio-opaque markers can be made of any densely packed material that creates a discernable feature on a fluoroscopic image. Examples include platinum, a platinum-iridium alloy, tungsten, barium, etc. The markers may be placed on the outside of the catheter shaft tube, or between the inner and outer layers, as shown in Figure 6.

The markers may be in various shapes and forms, including solid bands, C-shaped bands, foil ribbons, pad printed material in a viscous liquid carrier, injection molded, or applied using non-solid deposition (such as vapor deposition). Some formations are self-fixing to the inner member, while others must be accompanied by a secondary process to secure their location. Such processes include using glue (cyanoacrylate, UV curable, solvent bonding, etc.), crimping, swaging, roiling, or heat fusing.

The radio-opaque markers can be placed between the inner liner and braid layer, between the braid layer and outer layer, or on the outside of the outer layer. Their location will determine the sequence in which they are applied in the processes above. In the preferred embodiment they are placed between the braid layer and outer layer.

A proximal portion of the catheter shaft may also be reinforced with a hypotube component. The hypotube may have a flexibility that changes in specific areas. For example, the distal end of the hypotube may be more flexible than its proximal portion. The hypotube may be made of metal which is selected to be biocompatible, such as for example stainless steel. Other acceptable metals may include nitinol, stainless steel, high durometer polymer, or composite materials such as for example fiber glass, carbon fiber, etc.

The hypotube may be assembled over the outside of the outer layer with a close clearance, longitudinally aligned to the proximal end of the inner member. If desired, a strain relief can be created near its distal end to moderate the change in flexibility between the hypotube reinforcement and the polymer tube. This flexibility transition can be created using a spiral cut pattern at the distal end of the hypotube.

### Methods:

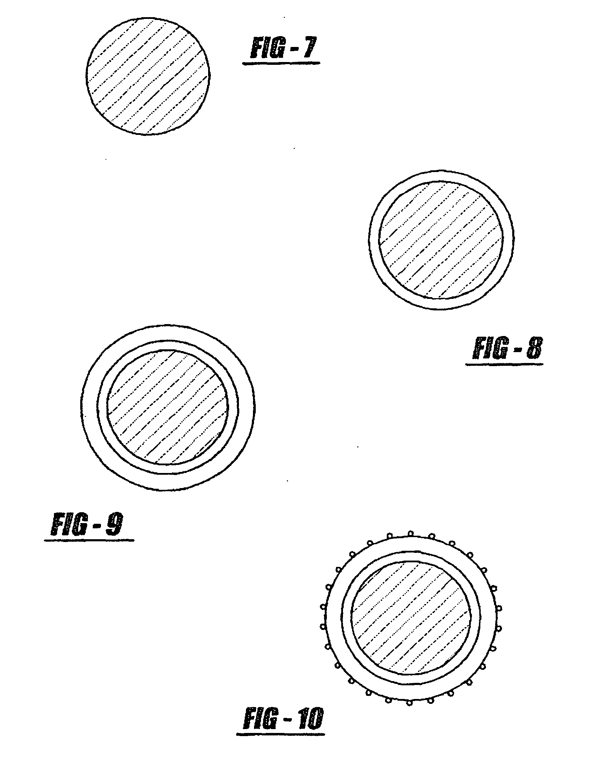

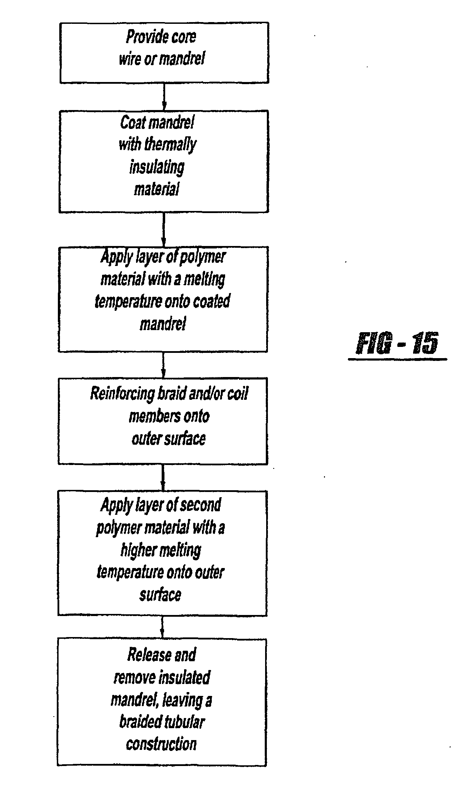

Figure 15 depicts a schematic flow chart of one possible method of making a catheter shaft tube according to the principles of the present inventions. A complementary set of cross-sections are shown in Figures 7-12, which illustrate various stages of an example process of the present inventions.

As shown in Figure 7, a core wire or mandrel serves as a foundation or substrate, around which the catheter shaft tube is built up. After the multi-layer tube is fully formed, the core wire will be removed, leaving the catheter shaft tube. The size of the core wire will determine the size of the catheter shaft tube lumen, when the core wire is eventually removed. Various materials may be used for the core wire, including stainless steel or copper, which may be silver-coated.

As shown in Figure 8, the core wire is coated with any suitable thermally insulating material, which may be applied by spraying or dip coating.

As shown in Figure 9, an inner polymer layer is applied to the core wire by any of several methods: wire mandrel extrusion, dipping or spraying using solvents and/or heat to create a solution of the polymer material, fuse-down techniques such as those employing shrink tubing, or other deposition techniques. According to some embodiments of the present inventions, the purpose of the inner polymer layer is to provide a lubricious inner surface for the lumen of the resulting tube, rather than to substantially contribute to the catheter shaft performance and structural properties. Accordingly, the inner polymer layer may be very thin, for example 0.0127 mm (0.0005 inch) or less. One possible material for the inner polymer layer is high-density polyethylene (HDPE), but many other suitable material choices are possible.

As shown in Figure 10, reinforcement is added, which may be of any desired configuration, such as a braid or coil. The reinforcement is applied to the outside of the inner polymer layer.

In the case of a braid, the braider can he arranged in a horizontal, vertical, or suitable other configuration. The braid or coil wire may be round stainless steel wire of a small diameter, such as for example 0.0762 mm (0.003 inch) or less. Of course, many material strands can be used, such as wire with a flat or other profile, different metal materials, natural fibers, polymers, synthetic fibers (Kevlar, LCP's, carbon, etc.), ceramics, and similar selections. Any number of braid wires can be applied simultaneously with the braider, ranging from 1 wire wound clockwise and 1 wire counterclockwise (referred to as "1 over 1"), up to the mechanical limit of a braider's capacity (such as for example 32 over 32).

The pitch of the reinforcement, which may be measured by the number of wire crossings per inch, called "pics," may be varied over the length of the catheter shaft tube. Thus, the proximal end of the tube may have a higher pitch (and lower pic count), creating a region of higher pushability and greater bending stiffness. Likewise, the distal end may have a lower pitch (and higher pic count), to creating a region of higher flexibility. In between these regions may be a transition zone where the pitch varies from a proximal pitch to a distal pitch. The pitch in this transition zone can change with a linear ramp, a step function with several regions, in a non-linear fashion such as an exponential curve, or some other slope that will produce the desired properties.

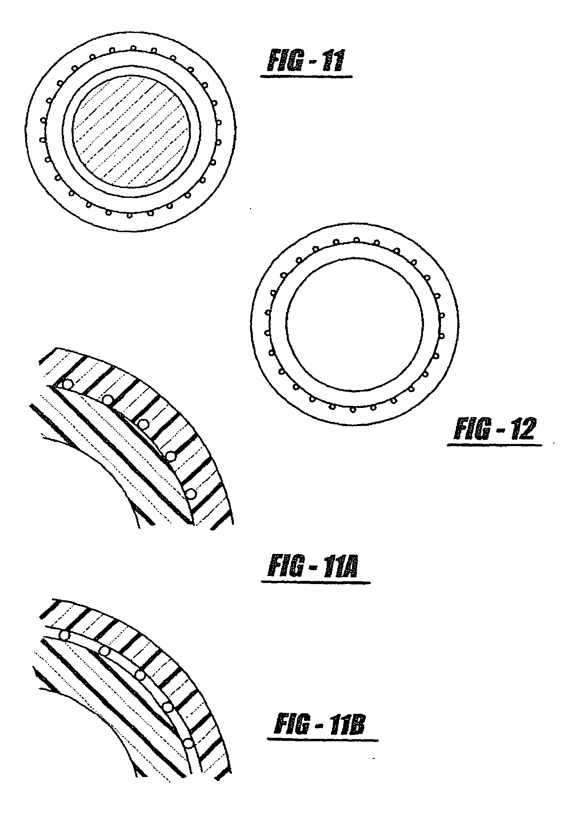

As shown in Figure 11, the outer polymer layer is applied to the existing subassembly. The material of the outer polymer layer may be poly-ether block amide (PEBA), and could also be a polyamide such as for example nylon, a polyimide, a fluoropolymer such for example (e.g. FEP, PTFE, etc.), a polyester, a polyolefin (e.g. HDPE. LDPE. PET, etc.), or similar polymers. The outer polymer layer is often designed to be thicker than the inner polymer layer, and may be in the range of 0.0254 - 0.125 mm (0.001-0.005 inch) thick.

The outer polymer layer can be comprised of two or more longitudinal segments that have different material properties. In one embodiment for example, there are three segments of differing flexibilities or durometers, with the proximal segment having the highest durometer, the distal segment having the lowest durometer and greatest flexibility, and the middle segment having an intermediate durometer. The selection and arrangement of material segments can help optimize catheter performance to complement the arrangement of other components: the inner polymer layer, reinforcement, and any other catheter components such as for example a hypotube or an outer polymer tube.

The construction method of the outer polymer layer will depend on how many longitudinal segments are selected. If the outer polymer layer will be only one segment the whole tube having the same outer polymer layer, then possible methods include: wire mandrel extrusion (in which the subassembly of coated core wire, inner polymer liner, and reinforcement act as a "wire mandrel"); dip coating or spraying using solvents and/or heat to create a solution of the material; or fuse-down techniques such as for example those employing shrink tubing; or other deposition techniques.

If the outer polymer layer will have two or more longitudinal segments, then possible methods include: fuse-down techniques such as for example those employing shrink tubing; build-up techniques where more (or different) material is selectively placed in the longitudinal regions to have more pushability and bending stiffness: or other deposition techniques.

As shown in Figures 11A and 11B, the catheter shaft tube may be made such that the outer polymer layer fills the spaces between the reinforcement members, or spans them.

As shown in Figure 12. the coated core wire is then removed, leaving the desired composite catheter shaft tube.

## Claims

1. A method of making a catheter (10, 18) for medically treating a patient, the method comprising:
coating a core wire with a thermally insulating material;
applying an inner polymer layer (34, 40, 50) to the core wire;
applying one or more reinforcing members to an outer surface of the inner polymer layer (34, 40, 50);
applying an outer polymer layer (36, 42, 52) to the reinforcement layer( 38, 48, 54); and
releasing and removing the coated core wire to form a flexible tubular shaft (12, 22) having proximal and distal ends and defining a lumen extending from a proximal port to a distal port;
wherein the inner polymer layer (34, 40, 50) has a lower melting temperature than the outer polymer layer (36, 42, 52).

2. The method of Claim 1, wherein the reinforcing members are selected from the group of: braids, coils, hoops, woven members, and longitudinal members.

3. The method of Claim 1, wherein the reinforcing members are of materials selected from the group of: stainless steel, nitinol, Kevlar, Dyneema, metals, and glass fibers.

4. The method of Claim 1, wherein the polymer layers (34, 40, 50; 36, 42, 52) are selected from the group of: polyamides, nylons, polyimides, polyethylenes, polyurethanes, polyether block amides.

5. The method of Claim 1, wherein the inner (34, 40, 50) and outer (36, 42, 52) layers are of different polymer materials, the inner layer being lubricious.

6. The method of Claim 1 , wherein the tubular shaft (12, 22) has a braided reinforcement extending between the inner (34, 40, 50) and outer (36, 42, 52) layers, and an amount of braid per longitudinal distance changes from a proximal position to a distal position.

7. The method of Claim 1, wherein the outer layer (36, 42, 52) is a nylon.

8. The method of Claim 1, wherein the outer layer (36, 42, 52) is a polyether block amide.

9. The method of Claim 1, wherein the inner layer (34, 40, 50) is high-density polyethylene.

## Patentansprüche

1. Verfahren zum Herstellen eines Katheters (10, 18) zum medizinischen Behandeln eines Patienten, wobei das Verfahren Folgendes umfasst:
Beschichten eines Kerndrahtes mit einem thermisch isolierenden Material;
Aufbringen einer inneren Polymerschicht (34, 40, 50) auf den Kerndraht;
Aufbringen von einem oder mehreren Verstärkungselementen auf eine äußere Oberfläche der inneren Polymerschicht (34, 40, 50);
Aufbringen einer äußeren Polymerschicht (36, 42, 52) auf die Verstärkungsschicht (3 8, 48, 54); und
Lösen und Entfernen des beschichteten Kerndrahtes, um einen flexiblen, röhrenförmigen Schaft (12, 22) zu bilden, welcher ein proximales und ein distales Ende aufweist und ein Lumen definiert, welches sich von einem proximalen Durchlass zu einem distalen Durchlass erstreckt;
wobei die innere Polymerschicht (34, 40, 50) eine geringere Schmelztemperatur als die äußere Polymerschicht (36, 42, 52) aufweist.

2. Verfahren nach Anspruch 1, wobei die Verstärkungselemente ausgewählt sind aus der Gruppe aus Flechtelementen, Spulen, Ringen, Webelementen und Längselementen.

3. Verfahren nach Anspruch 1, wobei die Verstärkungselemente aus Materalien hergestellt sind, die ausgewählt sind aus der Gruppe aus rostfreiem Stahl, Nitinol, Kevlar, Dyneema, Metallen und Glasfasern.

4. Verfahren nach Anspruch 1, wobei die Polymerschichten (34, 40, 50; 36, 42, 52) ausgewählt sind aus der Gruppe aus Polyamiden, Nylons, Polyimiden, Polyethylenen, Polyurethanen, Polyetherblockamiden.

5. Verfahren nach Anspruch 1, wobei die innere (34, 40, 50) und äußere (36, 42, 52) Schicht aus unterschiedlichen Polymermaterialien hergestellt sind, wobei die innere Schicht schlüpfrig ist.

6. Verfahren nach Anspruch 1, wobei der röhrenförmige Schaft (12, 22) eine geflochtene Verstärkung aufweist, welche sich zwischen der inneren (34, 40, 50) und der äußeren (36, 42, 52) Schicht erstreckt, und wobei sich eine Flechtmenge pro longitudinalem Abstand von einer proximalen Position zu einer distalen Position ändert.

7. Verfahren nach Anspruch 1, wobei die äußere Schicht (36, 42, 52) ein Nylon ist.

8. Verfahren nach Anspruch 1, wobei die äußere Schicht (36, 42, 52) ein Polyetherblockamid ist.

9. Verfahren nach Anspruch 1, wobei die innere Schicht (34, 40, 50) ein hochdichtes Polyethylen ist.

## Revendications

1. Méthode de fabrication d'un cathéter (10, 18) pour le traitement médical d'un patient, la méthode comprenant :
➢ le revêtement d'un fil central avec un matériau thermiquement isolant ;
➢ l'application d'une couche interne de polymère (34, 40, 50) sur le fil central ;
➢ l'application d'un ou de plusieurs éléments de renforcement sur la surface externe de la couche interne de polymère (34, 40, 50) ;
➢ l'application d'une couche externe de polymère (36, 42, 52) sur la couche de renforcement (38, 48, 54) ; et
➢ le déblocage et le retrait du fil central revêtu pour former une gaine tubulaire flexible (12, 22) possédant une extrémité proximale et une extrémité distale et définissant une lumière se prolongeant à partir d'un orifice proximal vers un orifice distal ;
dans laquelle la couche interne de polymère (34, 40, 50) possède une température de fusion plus basse que la couche externe de polymère (36, 42, 52).

2. Méthode selon la revendication 1, dans laquelle les éléments de renforcement sont choisis dans le groupe suivant : les tresses, les spires, les anneaux, les éléments tissés et les éléments longitudinaux.

3. Méthode selon la revendication 1, dans laquelle les éléments de renforcement sont composés de matériaux choisis dans le groupe suivant : l'acier inoxydable, le nitinol, le kevlar, la Dyneema, les métaux et les fibres de verre.

4. Méthode selon la revendication 1, dans laquelle les couches de polymère (34, 40, 50 ; 36, 42, 52) sont choisies dans le groupe suivant : les polyamides, les nylons, les polyimides, les polyéthylènes, les polyuréthanes, les amides séquencés polyéthérés.

5. Méthode selon la revendication 1, dans laquelle la couche interne (34, 40, 50) et la couche externe (36, 42, 52) sont composées de matériaux polymères différents, la couche interne étant glissante.

6. Méthode selon la revendication 1, dans laquelle la gaine tubulaire (12, 22) possède un renforcement tressé se prolongeant entre la couche interne (34, 40, 50) et la couche externe (36, 42, 52), et une quantité de tresse par distance longitudinale change à partir d'une position proximale vers une position distale.

7. Méthode selon la revendication 1, dans laquelle la couche externe (36, 42, 52) est un nylon.

8. Méthode selon la revendication 1, dans laquelle la couche externe (36, 42, 52) est un amide séquencé polyéthéré.

9. Méthode selon la revendication 1, dans laquelle la couche interne (34, 40, 50) est un polyéthylène haute densité.
